Europäisches Patentamt

European Patent Office

Office européen des brevets

(19)

(11) **EP 0 939 773 B1**

(12) **EUROPEAN PATENT SPECIFICATION**

(45) Date of publication and mention
of the grant of the patent:
**03.04.2002  Bulletin 2002/14**

(51) Int Cl.[7]: **C08B 37/14**, C08B 37/06,
A61L 15/60, A21D 2/36,
A23L 1/052

(21) Application number: **97912356.9**

(22) Date of filing: **14.11.1997**

(86) International application number:
**PCT/GB97/03140**

(87) International publication number:
**WO 98/22513 (28.05.1998 Gazette 1998/21)**

(54) **PRODUCTION OF VEGETABLE GELS**

VERFAHREN ZUR HERSTELLUNG VON PFLANZLICHEN GELEN

PRODUCTION DE GELS VEGETAUX

(84) Designated Contracting States:
**DE ES FR GB IT**

(30) Priority: **21.11.1996  GB 9624204**
**28.08.1997  GB 9718072**

(43) Date of publication of application:
**08.09.1999  Bulletin 1999/36**

(73) Proprietor: **Cambridge Biopolymers Limited**
**Duxford, Cambridgeshire CB2 4FB (GB)**

(72) Inventor: **FITCHETT, Colin, Stanley**
**Cambridge CB1 3AJ (GB)**

(74) Representative: **Davies, Jonathan Mark et al**
**Reddie & Grose**
**16 Theobalds Road**
**London WC1X 8PL (GB)**

(56) References cited:
**EP-A- 0 126 394          EP-A- 0 338 452**
**WO-A-96/03440          WO-A-97/17492**
**GB-A- 2 261 671**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention relates to hemicellulose-based gels and viscous media, to processes for their production, to products containing such gels and/or viscous media and to various applications thereof. In particular, the present invention relates to an improved method for performing oxidative gelation of hemicelluloses which avoids the need for the addition of hydrogen peroxide.

Plant tissue, especially cell wall material, contains hemicelluloses. The term "hemicellulose" is a term of art used to embrace non-cellulosic, non-starch plant polysaccharides. The term therefore embraces *inter alia* pentosans, pectins and gums.

Some hemicelluloses are suitable as substrates for oxidative gelation ("gelling hemicelluloses"): such hemicelluloses often have substituents with phenolic groups which are cross-linkable with certain oxidizing agents.

Arabinoxylan and pectin constitute two particularly important classes of hemicellulose. Arabinoxylans consist predominantly of the pentoses arabinose and xylose, and are therefore often classified as pentosans. However, in many cases hexoses and hexuronic acid are present as minor constituents, and therefore they may also be referred to descriptively as heteroxylans.

The arabinoxylan molecule consists of a linear backbone of (1-4)-β-xylopyranosyl units, to which substituents are attached through 02 and 03 atoms of the xlosyl residues. The major substituents are single α-L-arabinofuranosyl residues. Single α-D-glucoronopyranosyl residues and their 4-O-methyl ethers are also common substituents.

Arabinoxylan preparations are usually heterogenous with respect to the ratio of xylose to arabinose (i.e. the degree of substitution) and in the pattern of substitution of the arabinosyl units along the (1-4)-β-xylan backbone.

Phenolic acid (including ferulic acid) and acetyl substituents occur at intervals along the arabinoxylan chains. These substituents to some extent determine the solubility of the arabinoxylan. Arabinoxylan preparations bearing phenolic (e.g. ferulic acid substituents) are referred to herein as "AXF", while those bearing acetyl substituents are designated "AXA". Similarly, preparation bearing both phenolic (e.g. ferulic acid) and acetyl substituents are hereinafter abbreviated to the designation "AXFA". Arabinoxylan preparations having few phenolic (e.g. ferulic acid) substituents are designated "AX": when the degree of substitution falls below that required for oxidative gelation, the arabinoxylan is designated a "non-gelling arabinoxylan" (a term which therefore embraces AX and AXA).

Pectins constitute another important class of hemicelluloses. As used herein and unless otherwise indicated, the term "pectin" is used *sensu lato* to define hemicellulose polymers rich in D-galacturonic acid. Many (but not all) are cell wall components. The term "pectin" is also used herein *sensu stricto* to define the so-called "true pectins", which are characterised by the presence of an O-(α-D-galacturonopyranosyl)-(1-2)-L-rhamnopyranosyl linkage within the molecule.

The pectins may be subcategorized on the basis of their structural complexity. At one extreme are "simple pectins", which are galacturonans. At the other extreme are "complex pectins" exemplified by rhamnogalacturonan II, which contains at least 10 different monosaccharide components in the main chain or as a components of branches. Pectins of intermediate complexity (herein referred to as "mesocomplex pectins" contain alternate rhamnose and galacturonic acid units, while others have branches of glucoronic acid linked to galacturonic acid.

Complex and mesocomplex pectins are made up of "smooth" regions (based on linear homogalacturonan) and "hairy" regions corresponding to the rhamnogalacturonan backbone with side-branches of varying length.

Certain pectins (for example, pectins obtainable from representatives of the plant family *Chenopodiaceae*, which include beets (e.g. sugar beet), spinach and mangelwurzels) are substituted to some extent with substituents derived from carboxylic acids (usually substituted cinnamic acids) containing phenolic groups. Such pectins may be oxidatively crosslinked to produce viscous solutions or gels via their phenolic substituents. This can be achieved by powerful oxidants (e.g. persulfate - see J. - F. Thibault *et alia*, in The Chemistry and Technology of Pectin, Academic Press 1991, Chapter 7, pages 119-133) or a combination of peroxidase and hydrogen peroxide (see Thibault *et alia, ibidem*). FR 2 545 101 A1 also describes the gelling of beet pectins using an oxidant (e.g. hydrogen peroxide) and an enzyme (peroxidase). Such pectins are referred to herein as "gelling pectins".

Sugar beet pectin is especially rich in arabinan. Arabinan contains β1, 5-linked arabinose in the backbone with α-(1->3) or α-(1->2) - linked arabinose residues, whereas arabinogalactan contains β-1, 4-linked galactose in the backbone, with α-(1->3) or α-(1->2) linked arabinose residues. Ferulyl substituents are linked to the arabinose and/or the galactose in the arabinan and arabinogalactan side-branches of the rhamnogalacturonan part. The "ferulic acid" content varies according to the extraction method, but is often about 0.6%.

Beet pectins obtained by processes which partially remove arabinose residues may exhibit improved gelling properties. Thus, procedures involving mild acid treatment and/or treatment with an α-arabinofuranosidase will improve the gelling properties of the pectin (see F. Guillon and J. -F. Thibault, *ibidem*). Such pectins are hereinafter referred to as "treated pectins". Hemicelluloses are complex mixtures of noncellulosic cell wall polysaccharides, including pentosans such as arabinoxylans. Convenient sources of hemicelluloses include cereals (such as maize, barley, wheat, oats, rice), pulses (e.g. soya), legumes and fruit.

There are many known methods for fractionating plant material (such as testaceous or cell wall material) to produce hemicellulose and cellulose fractions. Such methods usually involve alkali or water extraction to yield insoluble cellulose and soluble hemicellulose fractions, followed by separation. The soluble extract is then often neutralized (or acidified) to precipitate hemicelluloses. Organic solvents are also commonly used instead of (or in addition to) acidification to precipitate further hemicellulose fractions.

Aqueous extracts of many hemicellulose fractions are known to form gels (or viscous media) when treated with oxidizing agents. The phenomenon is known as "oxidative gelation" in the art, but the term is used herein in a somewhat broader sense to include the case where viscous solutions are produced rather than true gels. This reflects the fact that oxidative gelation is a progressive phenomenon which may be controlled to vary the degree of gelation to the extent that hard, brittle gels are formed at one extreme and slurries, gravies or viscous liquids at the other.

The biochemical basis of the gelling process is not yet fully understood. However, gel formation and/or viscosity increases are thought to arise (at least in part) from cross linking within and/or between macromolecular components of the hemicellulose mediated by ferulic acid residues (for example, involving diferulate generated by oxidative coupling of the aromatic nucleus of ferulic acid). These ferulic acid residues occur on arabinoxylans present in the hemicellulose. Extensive hydrolysis (by e.g. harsh alkaline treatments) is known to strip the ferulic acid residues from the bulk pentosans, and so hemicelluloses for use as starting materials in the production of gels or viscous solutions are usually extracted by water (particularly hot water) or mild alkali extraction.

As used herein (and as is usual in the art), the terms "ferulic acid" and "ferulate" are used *sensu lato* encompass ferulyl (often denoted feruloyl) groups (i.e. 4-hydroxy-3-methoxy-cinnamyl groups) and derivatives (particularly oxidized derivatives) thereof.

Only a few oxidizing agents are known to have the ability to induce gelation, and these include hydrogen peroxide (usually in conjunction with a peroxidase), ammonium persulphate and formamidine disulphide.

WO 96/03440 describes the use of an oxidase (preferably a laccase) for promoting oxidative gelation of *inter alia* arabinoxylans. However, laccase may not be acceptable for use in certain food applications, is relatively expensive and the supply is limited. Moreover, oxidases such as laccase are relatively weak oxidation-promoters, and the range of different gel strengths obtainable by the use of such enzymes is limited. Indeed it is possible that the crosslinking achieved through the use of laccase and other oxidases differs fundamentally from that mediated by e.g. hydrogen peroxide, so that the gels may differ significantly in structure from those produced by other forms of oxidative gelation.

WO 93/10158 describes oxidative gelation of hemicellulosic material using an oxidizing system comprising a peroxide (such as hydrogen peroxide) and an oxygenase (such as a peroxidase). However, hydrogen peroxide is inconvenient as a reagent in industrial-scale processes, and is potentially dangerous.

WO 97/27221 discloses a method for causing gelling or increase of viscosity of an aqueous material containing a pectic material comprising treating the aqueous medium with a carboxylic ester hydrolase, an oxidase and/or a peroxidase in the presence of an oxidizing agent suitable for use with the oxidase and/or peroxidase.

There is therefore a need for alternative methods of promoting oxidative gelation which avoid the aforementioned problems.

Thus, according to the present invention there is provided a hemicellulosic material comprising an oxidase (e.g. glucose oxidase) and optionally a peroxidase (e.g. horse radish peroxidase) supplement, with the proviso that the hemicellulosic material is not a pectin.

The hemicellulose/hemicellulosic material for use in the invention may be any hemicellulose meeting the definition set out earlier. In particular, the hemicellulose may be an arabinoxylan, heteroxylan or pectin. In addition, the hemicellulose for use in the processes of the invention may be a synthetic hemicellulose (i.e. a structural analogue of a naturally-occurring hemicellulose synthesised *in vitro* by any chemical/enzymic synthesis or modification).

Thus, any non-cellulosic, non-starch plant polysaccharides may be used in the process of the invention. Thus, the processes of the invention find application in the processing *inter alia* of pentosans, pectins and gums.

Some hemicelluloses are suitable as substrates for oxidative gelation ("gelling hemicelluloses"): such hemicelluloses often have substituents with phenolic groups which are cross-linkable with certain oxidizing agents. These "gelling" hemicelluloses are particularly preferred for use in the invention. Non-gelling hemicelluloses may be first derivitized with phenolic (e. g. ferulic) acid groups prior to use in the invention.

Arabinoxylans, heteroxylans and pectins may also be used. Of the arabinoxylans, particularly preferred are AXFA, AXF, AXA and AX.

Also suitable for use in the invention are pectins, including the true pectins, simple pectins, complex pectins, mesocomplex pectins and gelling pectins (e.g. those obtainable from representatives of the plant family *Chenopodiaceae*, which include beets (e.g. sugar beet), spinach and mangelwurzels). Particularly preferred is sugar beet pectin (for example in the form of sugar beet pulp). Also useful in the invention are treated pectins (as hereinbefore defined).

The hemicellulosic material may be obtained by any of the standard techniques known in the art for obtaining hemicelluloses suitable as starting materials for oxidative gelation. Preferably, the hemicelluloses are obtained by any of the processes described in WO 93/10158.

As used herein, the term "supplement" as applied to any specified enzyme activity is intended to embrace not only the case where an appropriate enzyme preparation is added during production, but also encompasses the case where endogenous enzyme activity is activated, enhanced, induced or derepressed by any treatment (e.g. chemical or physical treatment) of the hemicellulosic material. Thus, the hemicellulosic material of the invention exhibit supplemental oxidase (and optionally peroxidase) activity howsoever achieved (so long as the level(s) of enzyme activity are sufficient e.g. to promote oxidative gelation), and are not essentially limited to hemicellulose preparations which have been prepared in any particular way.

Preferably, however, the enzyme supplement is added isolated enzyme having the desired activity. The level of purity and/or specificity is not crucial to the practise of the invention, so long as oxidase and/or peroxidase levels are elevated to levels sufficient to promote oxidative gelation under appropriate conditions.

As used herein, the term peroxidase denotes an enzyme which catalyses the general reaction:

$$H_2O_2 + H_2A \Rightarrow 2H_2O + A$$

where $H_2A$ is any oxidisable substrate. Without wishing to be bound by any theory, it is thought that in the case of oxidative gelation the substrate is the polysaccharide/ferulic acid complex, so resulting in crosslinking between the oxidized ferulic acid components via formation of a new C-C bond and the production of diferulic acid.

Preferred according to the present invention is peroxidase EC 1.11.1.7 (e.g. horse radish peroxidase). Alternatively, naturally occurring peroxidase activity endogenous to the hemicellulose material may be exploited according to the invention.

As used herein, the term oxidase denotes an enzyme which catalyses the general reaction:

$$O_2 + AH_2 \Rightarrow H_2O_2 + A$$

where $AH_2$ is glucose and the enzyme glucose oxidase, the reaction is:

$$O_2 + H_2O + glucose \Rightarrow H_2O_2 + gluconic acid$$

Preferred according to the present invention is glucose oxidase EC 1.1.3.4. (e.g. A. niger as source. Other oxidases which are suitable for use in the invention include amino acid oxidases, diamine oxidases and xanthine oxidase.

The gelation system of the invention avoids the dangers associated with excess of hydrogen peroxide (which carries a risk of explosion): in the gels of the invention a "negative feedback" loop ensures that if temperature rises due to excessive hydrogen peroxide production then the enzymes producing the hydrogen peroxide are progressively denatured as the temperatures rises, so limiting the production of further hydrogen peroxide.

The gellation system of the invention also has further important and unexpected advantages which *inter alia* permit the formulation of self-felling powders or solutions.

The material of the invention may further comprising an oxidase substrate (e.g. glucose) supplement. If glucose is used, then this has the ancillary advantage of acting as a dispersant. Alternatively, endogenous substrates naturally present in the hemicellulose may be exploited.

The hemicellulosic material may be derived from any of a wide range of different starting materials. Suitable starting materials containing hemicellulose for use in the invention typically include plant material of various kinds and any part or component thereof.

Plant materials useful as a starting material in the invention include the leaves and stalks of woody and nonwoody plants (particularly monocotyledonous plants), and grassy species of the famile Gramineae. Particularly preferred are gramineous agricultural residues, i.e. the portions of grain-bearing grassy plants which remain after harvesting the seed. Such residues include straws (e.g. wheat, oat, rice, barley, rye, buckwheat and flax straws), corn stalks, corn cobs and corn husks.

Other suitable starting materials include grasses, such as prairie grasses, gamagrass and foxtail. Other suitable sources include dicotyledonous plants such as woody dicots (e.g. trees and shrubs) as well as leguminous plants.

Another preferred source are fruits, roots and tubers (used herein in the botanical sense). The term "fruit" includes the ripened plant ovary (or group thereof) containing the seeds, together with any adjacent parts that may be fused with it at maturity. The term "fruit" also embraces simple dry fruits (follicles, legumes, capsules, achenes, grains, samaras and nuts (including chestnuts, water chestnuts, horsechestnuts etc.)), simple fleshy fruits (berries, drupes, false berries and pomes), aggregate fruits and multiple fruits. The term "fruit" is also intended to embrace any residual or modified leaf and flower parts which contain or are attached to the fruit (such as a bract). Encompassed within this meaning of fruit are cereal grains and other seeds. Also contemplated for use as starting materials are fruit components, including bran, seed hulls and culms, including malt culms. "Bran" is a component of cereals and is defined as a fraction obtained during the processing of cereal grain seeds and comprises the lignocellulosic seed coat as separate from the flour or meal. Other suitable component parts suitable as starting materials include flours and meals (particularly cereal flours and meals, and including nonwoody seed hulls, such as the bracts of oats and rice).

The term "root" is intended to define the usually underground portion of a plant body that functions as an organ of absorption, aeration and/or food storage or as a means of anchorage or support. It differs from the stem in lacking nodes, buds and leaves. The term "tuber" is defined as a much enlarged portion of subterranian stem (stolon) provided with buds on the sides and tips.

Preferred lignocellulosic starting materials include waste stream components from commercial processing of crop materials such as various beets and pulps thereof (including sugar beet pulp), citrus fruit pulp, wood pulp, fruit rinds, nonwoody seed hulls and cereal bran. Suitable cereal sources include maize, barley, wheat, oats, rice, other sources include pulses (e. g. soya), legumes and fruit.

Other suitable starting materials include pollen, bark, wood shavings, aquatic plants, marine plants (including algae), exudates, cultured tissue, synthetic gums pectins and mucilages.

Particularly preferred as a starting material is testaceous plant material, for example waste testaceous plant material (preferably containing at least about 20% of arabinoxylan and/or glucoronoarabinoxylan).

The starting material may be treated directly in its field-harvested state or (more usually) subject to some form of pre-processing. Typical pre-processing steps include chopping, grinding, cleaning, washing, screening, sieving etc.

Preferably, the starting material is in a substantially ground form having a particle size of not more than about 100 microns. It may be air classified or sieved (for example to reduce the level of starch). Alternatively, or in addition, the starting material may be treated with enzymes to remove starch (e.g. alpha- and/or beta-amylase). The starting material may also be pre-digested with a carbohydrase enzyme to remove β-glucan.

Suitable washing treatments include washing with hot water or acid (e.g. at a pH of 3-6, e.g. about 5). This at least partially separates protein. Other pre-treatments include protease treatment.

The hemicellulosic material may, for example, be obtained from cereal husk or bran, or legumes, e.g. from maize, wheat, barley, rice, oats or malt, though any source of hemicellulose may be used in the invention so long as it is subject to at least some degree of oxidative gelation.

Preferably, the hemicellulosic material comprises a pentosan, e.g. a water soluble or alkali soluble pentosan fraction. Particularly preferred are materials wherein the pentosan comprises arabinoxylan, for example arabinoxylan ferulate. In one preferred embodiment, the hemicellulose of the invention consists (or consists essentially) of arabinoxylan ferulate.

The material of the invention preferably takes the form of a powder, for example a substantially anhydrous powder. Powders according to the invention preferably contain a dispersant (e.g. glucose or maltodextrin). Such powders are conveniently formulated so as to be self-gelling on the addition of water in the presence of air, for example being formulated to contain oxidase, oxidase substrate (e.g. glucose) and optionally peroxidase supplements.

The invention also contemplates the material as described herein in the form of an aqueous solution. For some applications, such aqueous solutions are preferably oxygen free and packaged in containers which effectively exclude oxygen. Such solutions may be formulated so as to be is self-gelling on exposure to oxygen (e.g. the oxygen in ambient air), for example being formulated to contain oxidase, oxidase substrate (e.g. glucose) and optionally peroxidase supplements.

Also contemplated by the invention are gels or viscous media comprising the material of the invention which has been oxidatively gelled. Such gels or viscous media may comprise (or consist essentially of) cross linked arabinoxylan.

The invention also contemplates a process for preparing a gel or viscous medium comprising the step of oxidatively gelling the materials of the invention, for example by adding water to the anhydrous self-gelling powders or by exposing the oxygen free solutions to air or oxygen.

In another aspect, the invention contemplates a process for effecting oxidative gelation of a hemicellulosic material comprising the step of promoting the generation of hydrogen peroxide *in situ* by redox enzymes.

The redox enzymes preferably comprise an oxidase (e.g. glucose oxidase) and a peroxidase (e.g. horse radish peroxidase), which are preferably present as supplements in the hemicellulosic material.

According in this aspect of the invention, the process may comprise the steps of supplementing a hemicellulosic material with an oxidase and optionally an oxidase substrate and/or a peroxidase. The generation of hydrogen peroxide is then preferably promoted by:

    (a) providing oxygen to the material (e.g. by generating oxygen *in situ*); and/or
    (b) providing water to the material; and/or
    (c) providing oxidase substrate to the material (e.g. by generating substrate *in situ*); and/or
    (d) activating one or more of the redox enzymes (e. g. chemically or physically), wherein the provision of oxygen or substrate may be by controlled release or generation *in situ*, for example triggered generation or release by heat, irradiation or chemical treatment.

Where the oxygen is provided by triggering chemical production *in situ*, the invention finds particular application in retort cooking when the gel can be induced to form only on heating.

The invention also contemplates a gel or viscous medium produced by (or obtainable by) any of the processes of the invention.

In another aspect, the invention contemplates a process for producing a hemicellulosic material comprising the step of supplementing a hemicellulose with an oxidase (e.g. glucose oxidase) and optionally a peroxidase, (e.g. horse radish peroxidase), and also contemplates materials produced by (or obtainable by) such a process.

The hemicellulose products (i.e. the gels, dehydrated gels, rehydrated dehydrated gels, gelling (but ungelled) hemicelluloses and viscous liquids of the invention find a variety of applications various therapeutic, surgical, prophylactic, diagnostic and cosmetic (e.g. skin care) applications.

For example, the aforementioned materials may be formulated as a pharmaceutical or cosmetic preparation or medical device, for example selected from: a wound plug, wound dressing, wound debriding system, controlled release device, an encapsulated medicament or drug, a lotion, cream (e.g. face cream), suppository, pessary, spray, artificial skin, protective membrane, a neutraceutical, prosthetic, orthopaedic, ocular insert, injectant, lubricant or cell implant matrix. The non-gelling, gelling and gelled hemicelluloses (e.g. AX, AXF and gelled AXF) are particularly useful as agents which maintain the integrity of the gut wall lining, and as agents for coating the luminal wall of the gastrointestinal tract. They may therefore fins particular application in animal feeds and in the treatment of gastrointestinal disorders.

In such embodiments the material, gel or viscous medium of the invention may further comprising an antibiotic, electrolyte, cell, tissue, cell extract, pigment, dye, radioisotope, label, imaging agent, enzyme, co-factor, hormone, cytokine, vaccine, growth factor, protein (e.g. a therapeutic protein), allergen, hapten or antigen (for e.g. sensitivity testing), antibody, oil, analgesic and/or antiinflammatory agent (e.g. NSAID).

Thus, the above-listed materials find application in therapy, surgery, prophylaxis or diagnosis, for example in the treatment of surface (e.g. skin or membrane lesions, e.g. burns, abrasions or ulcers). In a particularly preferred embodiment, the invention contemplates a wound dressing comprising the above listed materials of the invention, for example in the form of a spray. Such wound dressings are particularly useful for the treatment of burns, where their great moisture retaining properties help to prevent the wound drying out.

Particularly preferred for such application is a self-gelling liquid comprising gelling hemicellulose supplemented with glucose and peroxidase and/or oxidase enzymes which gels on contact with oxygen in the air. Such compositions can be provided in the form of oxygen-free liquids in airtight containers which can be sprayed onto the skin, whereupon the liquid gels after exposure to the air. Such composition may advantageously be formulated so as to produce a slight excess of hydrogen peroxide on exposure to oxygen, so that a sterilizing, antibacterial, bacteriostatic and/or cleansing effect is obtained which helps promote healing.

The invention also contemplates water absorbent nappies, diapers, incontinence pads, sanitary towels, tampons and panty liners comprising the above-listed materials, as well as domestic and industrial cleaning or liquid (e.g. water) recovery operations (e.g. in the oil industry).

Alternatively, the gels of the invention can be provided in the form of hydrated or dehydrated sheets or pellicles for application to various internal or external surfaces of the body, for example during abdominal surgery to prevent adhesions.

Other applications include enzyme immobilizing systems, brewing adjuncts and bread improvers.

The materials listed above also find application as a foodstuff, dietary fibre source, food ingredient, additive, lubricant, supplement or food dressing. Such products are preferably selected from crumb, alginate replacer, cottage cheeses, aerosol toppings, frozen yoghurt, milk shakes, ice cream, low calorie products such as dressings and jellies, batters, cake mixes, frozen chips, binders, gravies, pastas, noodles, doughs, pizza toppings, sauces, mayonnaise, jam, preserve, pickles, relish, fruit drinks, a clouding agent in drinks, syrups, toppings and confectionary (e.g. soft centres) petfood (wherein the gel e.g. acts as a binder), a flavour delivery agent, a canning gel, fat replacer (e.g. comprising macerated gel), a coating, a glaze, a bait, a binder in meat and meat analogue products (for example vegetarian products), an edible adhesive, a gelatin replacer or dairy product or ingredient (e.g. a yoghurt supplement).

When used as a fat replacer the gel of the invention is preferably macerated to optimize its mouthfeel and fat mimetic properties.

The ungelled (but gellable) hemicelluloses (e.g. AXF) find particular application as clouding agents (e.g. in drinks), as film forming agents (e.g. in moisture barriers), glazes, edible adhesives and other functional food ingredients.

The invention will now be described by reference to the following examples which are purely exemplary and which do not limit the scope of the invention in any way.

Example 1

1,0g of a maize-derived hemicellulosic powder prepared according to the processes described in WO 93/10158 was mixed with 0.5g of glucose and 20mg each of peroxidase and glucose oxidase (Sigma). The composition gelled at 2% in water within 5 min on shaking in air.

Example 2

0.3g of a composition prepared as described in Example 1 was mixed with 6g of Regent (heat-treated) wheat flour and dispersed as a batter (3g of flour mix in 9g of water). The product became a solid gel in about 10 min.

Example 3

1g of the flour mix prepared as described in Example 2 was mixed with a further 5g of Regent flour and dispersed as a batter (3g of flour mix in 9g of water). The product became a solid gel in about 30 min.

Example 4

1.0g of a maize-derived hemicellulosic powder prepared according to the processes described in WO 93/10158 was mixed with 0.25g of glucose and 10mg each of peroxidase and glucose oxidase (Sigma). The composition gelled at 2% in water in 8 min on shaking in air.

Example 5

1.0g of a maize-derived hemicellulosic powder prepared according to the processes described in WO 93/10158 was mixed with 0.125g of glucose and 5mg each of peroxidase and glucose oxidase (Sigma). The composition gelled at 2% in water in 45 min on shaking in air.

Example 6

1.0g of a maize-derived hemicellulosic powder prepared according to the processes described in WO 93/10158 was mixed with 0.063g of glucose and 2.5mg each of peroxidase and glucose oxidase (Sigma). The composition gelled at 2% in water after 2 hours on shaking in air.

**Claims**

1. A hemicellulosic material comprising an oxidase supplement, a peroxidase supplement and optionally an oxidase substrate supplement, with the proviso that the hemicellulosic material is not a pectin.

2. The material of claim 1 wherein:

   (a) the oxidase is glucose oxidase; and/or
   (b) the peroxidase is horse radish peroxidase;
   and/or
   (c) the oxidase substrate supplement is glucose.

3. The material of claim 1 or claim 2 wherein the hemicellulosic material is derived from cereal flour, husk or bran, or from legumes (e.g. from maize, wheat, barley, rice, oats or malt).

4. The material of any one of claims 1-3 wherein the hemicellulosic material comprises a pentosan, e.g. a water soluble or alkali soluble pentosan fraction.

5. The material of claim 4 wherein the pentosan comprises arabinoxylan, for example, arabinoxylan ferulate.

6. The material of claim 5 wherein the hemicellulosic material consists or consists essentially of arabinoxylan ferulate.

7. The material of any one of the preceding claims in the form of a powder, for example a substantially anhydrous powder and optionally a dispersant (e.g. glucose or maltodextrin).

8. The material of claim 7 which comprises oxidase, oxidase substrate (e.g. glucose) and optionally peroxidase supplements, the material being self-gelling on the addition of water.

9. The material of any one of claims 1-8 in the form of an aqueous solution.

10. The material of claim 9 which is substantially oxygen free.

11. The material of claim 10 which comprises oxidase, oxidase substrate (e.g glucose) and optionally peroxidase supplements and which is self-gelling on exposure to oxygen.

12. A gel or viscous medium comprising the material of any one of claims 1-11 which has been oxidatively gelled.

13. The gel of claim 12 wherein the material comprises or consists essentially of cross linked arabinoxylan.

14. The gel or viscous medium of claim 1 2 or 1 3 in dehydrated form.

15. The dehydrated gel or viscous medium of claim 1 4 in rehydrated form.

16. A process for preparing a gel or viscous medium comprising the step of oxidatively gelling the material of any one of claims 1-11, for example by adding water to the material of claim 8 or by exposing the material of any one of claims 9-11.

17. A process for effecting oxidative gelation of a hemicellulosic material comprising the step of promoting the generation of hydrogen peroxide *in situ* with redox enzymes, wherein the redox enzymes comprise an oxidase and a peroxidase.

18. The process of claim 17 wherein the peroxidase is horse radish peroxidase and/or the oxidase is glucose oxidase.

19. The process of claim 17 or 18 wherein the process comprises the steps of supplementing a hemicellulosic material with an oxidase and optionally an oxidase substrate and/or a peroxidase.

20. The process of any one of claims 17-19 wherein the generation of hydrogen peroxide is promoted by:

(a) providing oxygen to the material (e.g. by generation or release *in situ*); and/or
(b) providing water to the material; and/or
(c) providing oxidase substrate to the material (e.g. by generation or release *in situ*); and/or
(d) activating one or more of the redox enzymes (e.g. chemically or physically),

wherein the provision of oxygen or substrate may be by controlled release or generation *in situ*, for example by triggered generation or release by heat, irradiation or chemical treatment(s).

21. A gel or viscous medium produced by (or obtainable by) the process of any one of claims 16-20, with the proviso that the hemicellulosic material therein is not a pectin.

22. A process for producing a hemicellulosic material (for example a material according to any one of claims 1-11) comprising the step of supplementing a hemicellulose with an oxidase (e.g. glucose oxidase) and a peroxidase (e.g. horse radish peroxidase) supplement., with the proviso that the hemicellulosic material is not a pectin.

23. A material produced by (or obtainable by) the process of claim 22.

24. A pharmaceutical or cosmetic preparation or medical device comprising the material, gel, viscous medium, dehydrated gel/viscous medium of any one of the preceding claims, the preparation or device being for example selected from: a wound plug, wound dressing, controlled release device, an encapsulated medicament or drug, a lotion, cream, suppository, pessary, spray, artificial skin, protective membrane, a neutraceutical, prosthetic, orthopaedic, ocular insert, injectant, lubricant or cell implant matrix, optionally further comprising an antibiotic, analgesic and/or anti-inflammatory agent.

25. The material, gel or viscous medium of any one of the preceding claims for use in therapy, prophylaxis or diagnosis, for example in the treatment of skin lesions (e.g. burns, abrasions or ulcers).

26. A wound dressing comprising the material of claim 11, for example in the form of a spray.

27. A bread improver comprising the material, gel or viscous medium of any one of the preceding claims.

28. A foodstuff, dietary fibre source, food ingredient, additive, lubricant, supplement or dressing comprising the material of any one of claims 1-11, 23 or 25, the gel or viscous medium of any one of claims 12-14, 21 or 25, for example being selected from a petfood (wherein the gel e.g. acts as a binder), a flavour delivery agent, a canning gel, fat replacer (e.g. comprising macerated gel of any one of the preceding claims), a coating, a glaze, a bait or a gelatin replacer.

29. A masking agent comprising the gel of any one of the preceding claims, for example for use in masking semiconductor wafers, etching plates or surfaces to be painted.

**Patentansprüche**

1. Hemicellulosematerial, umfassend einen Oxidasezusatz, einen Peroxidasezusatz und bei Bedarf einen Oxidasesubstratzusatz, unter der Voraussetzung, dass das Hemicellulosematerial kein Pektin ist.

2. Material nach Anspruch 1, bei dem:

(a) die Oxidase Glucoseoxidase ist; und/oder
(b) die Peroxidase Meerrettichperoxidase ist; und/oder
(c) der Oxidasesubstratzusatz Glucose ist.

3. Material nach Anspruch 1 oder Anspruch 2, bei dem das Hemicellulosematerial von Getreidemehl, Hülse oder Kleie, oder von Gemüse abgeleitet ist (z.B. von Mais, Weizen, Gerste, Reis, Hafer oder Malz).

4. Material nach einem der Ansprüche 1-3, bei dem das Hemicellulosematerial ein Pentosan, z.B. eine wasserlösliche oder alkalilösliche Pentosanfraktion umfasst.

**5.** Material nach Anspruch 4, bei dem das Pentosan Arabinoxylan wie beispielsweise Arabinoxylanferulat umfasst.

**6.** Material nach Anspruch 5, bei dem das Hemicellulosematerial ganz oder im Wesentlichen aus Arabinoxylanferulat besteht.

**7.** Material nach einem der vorherigen Ansprüche in der Form eines Pulvers, z.B. ein im Wesentlichen wasserfreies Pulver und bei Bedarf ein Dispergiermittel (z.B. Glucose oder Maltodextrin).

**8.** Material nach Anspruch 7, das Oxidase, Oxidasesubstrat (z.B. Glucose) und bei Bedarf Peroxidasezusätze umfasst, wobei das Material bei Zugabe von Wasser selbstgelierend ist.

**9.** Material nach einem der Ansprüche 1-8 in der Form einer wässrigen Lösung.

**10.** Material nach Anspruch 9, das im Wesentlichen sauerstofffrei ist.

**11.** Material nach Anspruch 10, das Oxidase, Oxidasesubstrat (z.B. Glucose) und bei Bedarf Peroxidasezusätze umfasst und das bei Kontakt mit Sauerstoff selbstgelierend ist.

**12.** Gel oder viskoses Medium, umfassend das Material nach einem der Ansprüche 1-11, das oxidativ geliert wurde.

**13.** Gel nach Anspruch 12, bei dem das Material vernetztes Arabinoxylan umfasst oder im Wesentlichen daraus besteht.

**14.** Gel oder viskoses Medium nach Anspruch 12 oder 13 in dehydrierter Form.

**15.** Dehydriertes Gel oder viskoses Medium nach Anspruch 14 in rehydrierter Form.

**16.** Verfahren zur Herstellung eines Gels oder viskosen Mediums, umfassend den Schritt des oxidativen Gelierens des Materials nach einem der Ansprüche 1-11, beispielsweise indem dem Material nach Anspruch 8 Wasser zugegeben oder das Material nach einem der Ansprüche 9-11 Sauerstoff ausgesetzt wird.

**17.** Verfahren zum Bewirken einer oxidativen Gelierung eines Hemicellulosematerials, umfassend den Schritt des Förderns der Erzeugung von Wasserstoffperoxid *in situ* mit Redox-Enzymen, wobei die Redox-Enzyme eine Oxidase und eine Peroxidase umfassen.

**18.** Verfahren nach Anspruch 17, bei dem die Peroxidase Meerrettichperoxidase ist und/oder die Oxidase Glucoseoxidase ist.

**19.** Verfahren nach Anspruch 17 oder 18, wobei das Verfahren die Schritte des Ergänzens eines Hemicellulosematerials mit einer Oxidase und bei Bedarf mit einem Oxidasesubstrat und/oder mit einer Peroxidase umfasst.

**20.** Verfahren nach einem der Ansprüche 17-19, bei dem die Erzeugung von Wasserstoffperoxid gefördert wird durch:

(a) Zuführen von Sauerstoff zu dem Material (beispielsweise durch Erzeugung oder Freisetzung *in situ*) und/oder
(b) Zuführen von Wasser zu dem Material; und/oder
(c) Zuführen von Oxidasesubstrat zu dem Material (z.B. durch Erzeugung oder Freisetzung *in situ*); und/oder
(d) Aktivieren von einem oder mehreren der Redox-Enzyme (z.B. chemisch oder physikalisch),

wobei die Zuführung von Sauerstoff oder Substrat durch eine kontrollierte Freisetzung oder Erzeugung *in situ* erfolgen kann, z.B. durch eine durch Wärme, Strahlung oder chemische Behandlung(en) ausgelöste Erzeugung oder Freisetzung.

**21.** Gel oder viskoses Medium, hergestellt mit (oder erhaltbar mit) dem Verfahren nach einem der Ansprüche 16-20, unter der Voraussetzung, dass das Hemicellulosematerial kein Pektin ist.

**22.** Verfahren zur Herstellung eines Hemicellulosematerials (z.B. eines Materials nach einem der Ansprüche 1-11), umfassend den Schritt des Ergänzens einer Hemicellulose mit einem Oxidase- (z.B. Glucoseoxidase) und einem Peroxidase- (z.B. Meerrettichperoxidase) Zusatz unter der Voraussetzung, dass das Hemicellulosematerial kein Pektin ist.

**23.** Material, hergestellt mit (oder erhaltbar mit) dem Verfahren nach Anspruch 22.

**24.** Pharmazeutisches oder kosmetisches Präparat oder medizinisches Gerät, umfassend das Material, Gel, viskose Medium, dehydrierte Gel/viskose Medium nach einem der vorherigen Ansprüche, wobei das Präparat oder Gerät beispielsweise ausgewählt wird aus: einem Wundpropfen, einem Wundverband, einem Gerät zur kontrollierten Freisetzung, einem verkapselten Medikament oder Arzneimittel, einer Lotion, einer Creme, einem Suppositorium, einem Pessar, einem Spray, einer künstlichen Haut, einer Schutzmembran, einer neutrozeutischen, prothetischen, orthopädischen, Okulareinsatz-, Einspritzmittel-, Schmierstoff- oder Zellenimplantatmatrix, bei Bedarf ferner ein Antibiotikum, Analgesikum und/oder ein Entzündungshemmmittel umfassend.

25. Material, Gel oder viskoses Medium nach einem der vorherigen Ansprüche für die Verwendung bei Therapie, Prophylaxe oder Diagnostik beispielsweise bei der Behandlung von Hautläsionen (z.B. Verbrennungen, Abschürfungen oder Geschwüren).

26. Wundverband, umfassend das Material nach Anspruch 11, z.B. in der Form eines Sprays.

27. Brotverbesserer, umfassend das Material, Gel oder viskose Medium nach einem der vorherigen Ansprüche.

28. Lebensmittel, Nahrungsfaserquelle, Lebensmittelingrediens, Additiv, Schmierstoff, Zusatz oder Verband, umfassend das Material nach einem der Ansprüche 1-11, 23 oder 25, das Gel oder viskose Medium nach einem der Ansprüche 12-14, 21 oder 25, beispielsweise ausgewählt aus einem Tierfuttermittel (bei dem das Gel beispielsweise als Bindemittel fungiert), einem Geschmacksstoff, einem Konservierungsgel, einem Fettersatz (z.B. mazeriertes Gel nach einem der vorherigen Ansprüche umfassend), einer Beschichtung, einer Glasur, einem Köder oder einem Gelatineersatz.

29. Maskierungsmittel, umfassend das Gel nach einem der vorherigen Ansprüche, z.B. für die Verwendung bei der Maskierung von Halbleiterwafern, Ätzplatten oder zu lackierenden Oberflächen.

## Revendications

1. Matière hémicellulosique comprenant un complément d'oxydase, un complément de peroxydase et facultativement un complément de substrat d'oxydase, à condition que la matière cellulosique ne soit pas une pectine.

2. Matière de la revendication 1 dans laquelle :

   (a) l'oxydase est de l'oxydase de glucose; et/ou
   (b) la peroxydase est de la peroxydase de raifort; et/ou
   (c) le complément de substrat d'oxydase est du glucose.

3. Matière de la revendication 1 ou 2 dans laquelle la matière hémicellulosique est dérivée de farine, d'enveloppes ou de son de céréales, ou de légumes (par ex. de maïs, de blé, d'orge, de riz, d'avoine ou de malt).

4. Matière de l'une quelconque des revendications 1-3 dans laquelle la matière hémicellulosique comprend un pentosane, par ex. une fraction pentosane soluble dans l'eau ou soluble dans les alcalis.

5. Matière de la revendication 4 dans laquelle le pentosane comprend de l'arabinoxylane, par exemple, du férulate d'arabinoxylane.

6. Matière de la revendication 5 dans laquelle la matière hémicellulosique consiste ou consiste essentiellement en férulate d'arabinoxylane.

7. Matière de l'une quelconque des revendications précédentes sous la forme d'une poudre, par exemple une poudre sensiblement anhydre et facultativement un dispersant (par ex. du glucose ou de la maltodextrine).

8. Matière de la revendication 7 qui comprend des compléments d'oxydase, de substrat d'oxydase (par ex. du glucose) et facultativement de peroxydase, la matière étant auto-gélifiante lors de l'addition d'eau.

9. Matière de l'une quelconque des revendications 1-8 sous la forme d'une solution aqueuse.

10. Matière de la revendication 9 qui est sensiblement sans oxygène.

11. Matière de la revendication 10 qui comprend des compléments d'oxydase, de substrat d'oxydase (par ex. du glucose) et facultativement de peroxydase et qui est auto-gélifiante à l'exposition à l'oxygène.

12. Gel ou milieu visqueux comprenant la matière de l'une quelconque des revendications 1-11 qui a été gélifiée par oxydation.

13. Gel de la revendication 12 dans lequel la matière comprend ou consiste essentiellement en arabinoxylane réticulé.

14. Gel ou milieu visqueux de la revendication 12 ou 13 sous forme déshydratée.

15. Gel ou milieu visqueux déshydraté de la revendication 14 sous forme réhydratée.

16. Procédé pour préparer un gel ou milieu visqueux comprenant l'étape consistant à gélifier par oxydation la matière de l'une quelconque des revendications 1-11, par exemple en ajoutant de l'eau à la matière de la revendication 8 ou en exposant la matière de l'une quelconque des revendications 9-11 à l'oxygène.

17. Procédé pour effectuer la gélification par oxydation d'une matière hémicellulosique comprenant l'étape consistant à favoriser la production de peroxyde d'hydrogène *in situ* avec des enzymes rédox, où les enzymes rédox comprennent une oxydase et une peroxydase.

18. Procédé de la revendication 17 dans lequel la peroxydase est de la peroxydase de raifort et/ou l'oxydase est de l'oxydase de glucose.

19. Procédé de la revendication 17 ou 18 dans lequel le procédé comprend les étapes consistant à compléter une matière hémicellulosique avec une oxydase et facultativement un substrat d'oxydase et/ou une peroxydase.

20. Procédé de l'une quelconque des revendications 17-19 dans lequel la production de peroxyde d'hydrogène est favorisée en :

> (a) fournissant de l'oxygène à la matière (par ex. production ou libération *in situ*); et/ou
> (b) fournissant de l'eau à la matière; et/ou
> (c) fournissant un substrat d'oxydase à la matière (par ex. par production ou libération *in situ*); et/ou
> (d) activant une ou plusieurs enzymes rédox (par ex. chimiquement ou physiquement),

> dans lequel la fourniture d'oxygène ou substrat peut se faire par libération contrôlée ou production *in situ*, par exemple par production ou libération déclenchée par la chaleur, l'irradiation ou un/des traitement(s) chimique(s).

21. Gel ou milieu visqueux produit par (ou pouvant être obtenu par) le procédé de l'une quelconque des revendications 16-20, à condition que la matière hémicellulosique dans celui-ci ne soit pas une pectine.

22. Procédé pour produire une matière hémicellulosique (par exemple une matière selon l'une quelconque des revendications 1-11) comprenant l'étape consistant à compléter une hémicellulose avec un complément d'oxydase (par ex. oxydase de glucose) et de peroxydase (par ex. peroxydase de raifort), à condition que la matière hémicellulosique ne soit pas une pectine.

23. Matière produite par (ou pouvant être obtenue par) le procédé de la revendication 22.

24. Préparation pharmaceutique ou cosmétique ou dispositif médical comprenant la matière, le gel, le milieu visqueux, le gel/milieu visqueux déshydraté de l'une quelconque des revendications précédentes, la préparation ou le dispositif étant par exemple sélectionné parmi : une mèche pour plaies, pansement pour plaies, dispositif à libération contrôlée, médicament ou drogue en capsule, une lotion, crème, suppositoire, pessaire, pulvérisation, peau artificielle, membrane de protection, un nutraceutique, un appareil prothétique, orthopédique, un implant oculaire, un produit injectable, un lubrifiant ou une matrice d'implant cellulaire, comprenant facultativement en outre un antibiotique, un analgésique et/ou un agent anti-inflammatoire.

25. Matière, gel ou milieu visqueux de l'une quelconque des revendications précédentes à utiliser en thérapie, prophylaxie ou diagnostic, par exemple dans le traitement de lésions de la peau (par ex. brûlures, abrasions ou ulcères).

26. Pansement pour plaies comprenant la matière de la revendication 11, par exemple sous forme d'une pulvérisation.

27. Améliorant de pain comprenant la matière, le gel ou le milieu visqueux de l'une quelconque des revendications précédentes.

28. Produit alimentaire, source de fibres alimentaires, ingrédient alimentaire, additif, lubrifiant, complément ou assaisonnement comprenant la matière de l'une quelconque des revendications 1-11, 23 ou 25, le gel ou le milieu visqueux de l'une quelconque des revendications 12-14, 21 ou 25, par exemple étant sélectionné parmi un aliment pour animaux domestiques (dans lequel le gel agit par ex. comme liant), un agent de sapidité, un gel de mise en conserve, un produit de remplacement de matière grasse (par ex. comprenant du gel macéré de l'une quelconque des revendications précédentes), un enrobage, un glaçage, un appât ou un produit de remplacement de gélatine.

29. Agent masquant comprenant le gel de l'une quelconque des revendications précédentes, par exemple à utiliser pour masquer des plaquettes semiconductrices, des plaques à graver ou des surfaces à peindre.